# EUROPEAN PATENT SPECIFICATION

(11) **EP 2 863 854 B1**
(45) Date of publication and mention of the grant of the patent: **02.01.2019**
(21) Application number: 13809200.2
(22) Date of filing: 26.06.2013
(51) Int. Cl.: A61B 17/08

(54) **WOUND CLOSURE DEVICE**
WUNDVERSCHLUSSVORRICHTUNG
DISPOSITIF DE FERMETURE DES PLAIES

(30) Priority: 26.06.2012 US 201261664463 P; 15.03.2013 US 201313843631
(43) Date of publication of application: 29.04.2015
(73) Proprietor: Weiser, Leslie Philip, Wellesley, MA 02481 (US)
(72) Inventor: Weiser, Leslie Philip, Wellesley, MA 02481 (US)
(74) Representative: FRKelly
(86) International application number: PCT/US2013/048005
(87) International publication number: WO 2014/004740

(56) References cited:
- EP-A2- 0 303 422
- WO-A1-93/04650
- US-A1- 2002 099 315
- US-A1- 2004 243 040
- US-A1- 2005 080 453
- US-A1- 2008 228 219
- US-A1- 2010 228 287
- US-B1- 6 293 281

## Description

### FIELD

The present teachings generally relate to wound closure devices, and more specifically to sutureless wound closure devices and methods.

### BACKGROUND

This application involves wound closures similar to the type described in U.S. Patent No. 7,981,136, issued on July 19, 2011, U.S. Patent Applications, Serial No. 10/884,837, filed July 2, 2004, Serial No. 10/412,967, filed April 14, 2003, and US Provisional Patent Application Nos. 60/873,643, filed December 8, 2006, and 60/934,248, filed June 12, 2007, all owned in common with the instant application.

Among the most commonly used methods for closing wounds caused by lacerations or surgical incisions are suturing and stapling. Both of these procedures are skin invasive, which can traumatize and compromise the integrity of the wound. They increase the possibility of infection, expose the surgeon, as well as the patient, to blood-borne disease, leave behind scar tracks, and require a follow-up visit for suture or staple removal.

As is well known, a cut that invades deeply into the tissue of the skin generally requires a mechanism for drawing the sides of a wound together to promote healing and to reduce the formation of scar tissue. Surgeons have become skilled in the various techniques of suturing to minimize the resulting blemish that occurs during the healing process. These methods have always generated issues of sterilization and the very nature of suturing requires a threshold of dexterity that escapes many care providers. This is particularly true in emergency situations, which call for immediate treatment to secure the wound for transport or until such time as proper surgery is available. Suturing, even by a skilled surgeon, punctures and stresses skin tissue causing scaring. In certain situations, such as in geriatric and pediatric applications, when patient's skin is either thin and fragile, using sutures can be impractical or impossible.

US2005080453 discloses methods for producing an interlaced device or composition which includes at least a first and a second interlaced element. Each interlaced element includes a mated first and second part, the first part having two termini and the second part having two termini, and a defined central void through which the other interlaced element passes.

US2010228287 discloses a device for adhering to the skin of a patient, suitable to make a skin incision there through, as well as to the use of such a device for allowing an incision or excision wound to be made through the said device, and for subsequent closing the wound.

US2008228219 discloses an elongated flexible base strip having its bottom surface coated with an adhesive material suitable for adherence to skin and constructed with bridging links spaced along the inner edge of the base strip and extending outwardly therefrom. The inner edge of the base strip is intended to be aligned adjacent to a lip of the wound being treated. Each of the bridging links has an adhesive coated section displaced from the inner edge.

US2004243040 discloses an elongated flexible base strip constructed with a bottom surface coated with an adhesive material. The base strip is constructed with bridging links that are spaced along the inner edge of the base strip and extend outward therefrom. Adhesive sections above and below the strip are exposed and a shield is provided to protect the exposed adhesive sections from above and below the base strip.

It is well recognized that a sutureless wound closure would be a great benefit in many situations. Accordingly, the present disclosure provides improved sutureless wound closure devices and methods which overcomes the above problems and others.

### SUMMARY

The present invention is defined by appended claim 1. Specific embodiments are set forth in the dependent claims.

In certain aspects the present teachings provide for a device for closing a wound. The device comprises a flexible base strip. The base strip has a bottom surface coated with an adhesive to adhere to one side of a wound, and a top surface opposite the bottom surface. The base also has an inner edge and an outer edge. The base strip also has a plurality of bridging links integrated with it in axially-spaced relation. The bridging links extending transversely from the inner edge of the base strip. Each bridging link can be moved about a hinge region between a first, storage position, in which the bridging links are folded over the top surface of the base strip, and a second, extended position, in which the bridging links extend outwardly from the inner edge of the base strip. Each bridging link has a first, engaging surface and a second surface opposite the engaging surface. The first engaging surface has an attachment region for attaching to another top surface of another base strip when that another base strip is placed on an opposite side of the wound and the bridging links are in the extended position. The hinge region of each bridging link comprises a partially flexible material capable of stabilizing the orientation of the bridging link in an intermediate angular position between the first and the second positions. This device may comprise a holding tab integrated with the outer edge of the base strip and extending outwardly a predetermined distance from the outer edge. The holding tab is substantially free from adhesive and may be used for holding. The base strip of the device may be comprised of, for example, but not limited to, a loop tape of a hook-and-loop type fastener substrate. The loop tape is positioned to present loops at least on a portion of the top surface of the base strip. Complementary bridging links may comprise, for example, of a hook tape of the hook-and-loop type fastener substrate. The hook tape is oriented to present hooks in the attachment regions of the bridging links. The bridging links of the device may include a pulling tab at their outer ends. These pulling tabs are substantially free from adhesive and may be used for holding. The base strip and the bridging of the device may also be made of polyurethane or, for example, but not limited to, a breathable unidirectionally elastic substrate. The bridging links of the device may have adhesive in a locking area on the second surface. When the bridging links are in the stored position the locking area may be used to releasably secure the second surface to the top surface of the base strip. The device may have transverse indicia on the top of the bottom surfaces of the base strip. The transverse indicia may be used for guiding separating the base strip into shorter fragments. The device may also be comprised of a holding film attached to the bottom surface of the base strip at a predetermined distance from the inner edge of the base strip and extending beyond the outer edge of the base strip.

In certain aspects the present teachings provide for another device for closing a wound. The device comprises some of or all of the following, a first closure strip removably attached to a lower support sheet, and a second closure strip removably attached to the lower support sheet. The first and second closure strips are in aligned, facing relation. Each of the first and second closure strips of the device includes an elongate, flexible base strip. The base strip has a bottom surface coated with an adhesive to adhere to a first side of a wound and a top surface opposite the bottom surface. The base strip has an inner edge and an outer edge. Each of the first and second closure strips include a plurality of bridging links connected to the base strip in axially-spaced relation. The bridging links extend transversely from the inner edge. Each of the bridging links are movable about a hinge region between a first, storage position in which the bridging links are folded over the top surface of the base strip, and a second, extended position in which the bridging links extend outwardly from the inner edge. Each bridging link has a first, engaging surface and a second surface opposite the engaging surface. The first engaging surface has an attachment region for attaching to another top surface of another base strip when that another base strip is placed on an opposite side of the wound and the bridging links are in their extended positions. The hinge region of each bridging link is comprised of a partially flexible material capable of stabilizing the orientation of the bridging links in an intermediate angular position between their first and second positions. Each bridging link of the device may further be comprised of a pulling tab at its outer end. The pulling tab is substantially free from adhesive and may be used for holding. The base strip and the bridging links of the device may comprise polyurethane or, for example, but not limited to, a breathable unidirectionally elastic substrate. Each bridging link may have adhesive in a locking area on the second surface. The locking area may be used to releasably secure the bridging link to the top surface of the base strip when the bridging link is in the storage position. The device may have a transverse indicia on the top surface or the bottom surface of the first or second closure strips. The transverse indicia may be used for guiding and/or separating the first or second closure strips into shorter fragments. The device may include a first holding film removably attached to the bottom surface of the first closure strip, between the first closure strip and the support sheet. The first holding film is attached at a predetermined distance from the inner edge of the first closure strip while extending beyond the outer edge of the first closure strip. The device may also include a second holding film removably attached to the bottom surface of the second closure strip, between the second closure strip and the support sheet. The second holding film is attached at a predetermined distance from the inner edge of the second closure strip while extending beyond the outer edge of the second closure strip. When the bridging links are in their storage position, the device may include a top film removably attached to the attachment regions of the bridging links. The support sheet of the device may have an indicia line between the first and second closure strips.

In certain aspects the present teachings provide for a method of closing a wound. The method includes some of or all of the following, selecting a wound closing device. The wound closing device comprises a first closure strip removably attached to a lower support sheet, and a second closure strip removably attached to the lower support sheet. The first and second closure strips are in aligned, facing relation. Each of the first and second closure strips of the device includes an elongate, flexible base strip. The base strip has a bottom surface coated with an adhesive to adhere to a first side of a wound and a top surface opposite the bottom surface. The base strip has an inner edge and an outer edge. Each of the first and second closure strips include a plurality of bridging links connected to the base strip in axially-spaced relation. The bridging links extend transversely from the inner edge. Each of the bridging links are movable about a hinge region between a first, storage position in which the bridging links are folded over the top surface of the base strip, and a second, extended position in which the bridging links extend outwardly from the inner edge. Each bridging link has a first, engaging surface and a second surface opposite the engaging surface. The first engaging surface has an attachment region for attaching to another top surface of another base strip when that another base strip is placed on an opposite side of the wound and the bridging links are in their extended positions. The hinge region of each bridging link comprises a partially flexible material capable of stabilizing orientation of the bridging links in an intermediate angular position between their first and second positions. Each bridging link of the device further comprises a pulling tab at its outer end. The pulling tab is substantially free from adhesive and may be used for holding. The device includes a first holding film removably attached to the bottom surface of the first closure strip, between the first closure strip and the support sheet. The first holding film is attached at a predetermined distance from the inner edge of the first closure strip while extending beyond the outer edge of the first closure strip. The device also includes a second holding film removably attached to the bottom surface of the second closure strip, between the second closure strip and the support sheet. The second holding film is attached at a predetermined distance from the inner edge of the second closure strip while extending beyond the outer edge of the second closure strip. The method further includes identifying a mammalian wound. The method includes a step of removing the first closure strip from the support sheet while holding the first holding film. The method includes a step of adhering an unprotected with the first holding film portion of the bottom surface of the first closure strip to skin on one side of the wound at a predetermined distance from one wound lip. The method includes a step of removing the first holding film from the bottom surface of the first closure strip while adhering the bottom surface of the first closure strip to the skin on the one side of the wound. The method includes a step of removing the second closure strip from the support sheet while holding the second holding film. The method includes a step of adhering an unprotected with the second holding film portion of the bottom surface of the second closure strip to skin on an opposite side of the wound at a predetermined distance from an opposite wound lip. The method includes a step of removing the second holding film from the bottom surface of the second closure strip while adhering the bottom surface of the second closure strip to the skin on the opposite side of the wound. The method includes a step of attaching the attachment region of at least one bridging link of the first closure strip to the top surface of the base strip of the second closure strip while holding the pulling tab of that bridging link of the first closure strip. The method includes a step of attaching the attachment region of at least one bridging link of the second closure strip to the top surface of the base strip of the first closure strip while holding the pulling tab of that bridging link of the second closure strip. And the method includes a step of leaving one bridging link in the intermediate position while manipulating another bridging link.

### BRIEF DESCRIPTION OF THE DRAWINGS

The invention may take form in various components and arrangements of components, and in various steps and arrangements of steps. The drawings are only for purposes of illustrating preferred embodiments and are not to be construed as limiting the invention.
**FIG. 1** illustrates a principal unit of wound closure device of the present teachings.
**FIG. 2** illustrates an extended wound closure strip of the present teachings.
**FIG. 2A** illustrates an example of a wound closure strip comprised of principal units without a locking area and having extended partially flexible components.
**FIG. 3** shows an illustration of an extended wound closure strip of the present teachings comprised of a plurality of principal wound closure units and lines marking approximate separations between units.
**FIG. 4** illustrates application of two wound closure strips of the present teachings to a wound.
**FIG. 5** illustrates an exploded view of a ready-for-use wound closure device of the present teachings.
**FIG. 5A** illustrates an exploded view of a ready-for-use wound closure device of the present teachings having extended holding strips.
**FIG. 6** illustrates an implementation of holding tabs on a wound closure strip of the present teachings.
**FIG. 7A** illustrates wound closure strip base holes allowing for strip expansion and shrinkage during wound healing.
**FIG. 7B** illustrates wound closure strip base slits allowing for strip expansion and shrinkage during wound healing.
**FIG. 8** illustrates wound closure strip base notches augmenting, for example, separating the strip into individual units.

### DETAILED DESCRIPTION

Although the present invention will be described with reference to the embodiments shown in the figures, it should be understood that the present invention may have many alternate forms.

In the course of describing the wound closure embodiments herein, the bottom of the closing device will refer to the surface that is intended to engage the skin and the upper side or top will refer to the side of a component that is facing away from the skin after application. Directions will be indicated according to the position of the wound being treated, for example, transverse shall refer to directions across the wound. The inner edge of the closing device shall refer to the side that is intended to be adjacent to the wound lip (or edge), and the outer edge shall refer to the side of the device that is intended to be away from the wound.

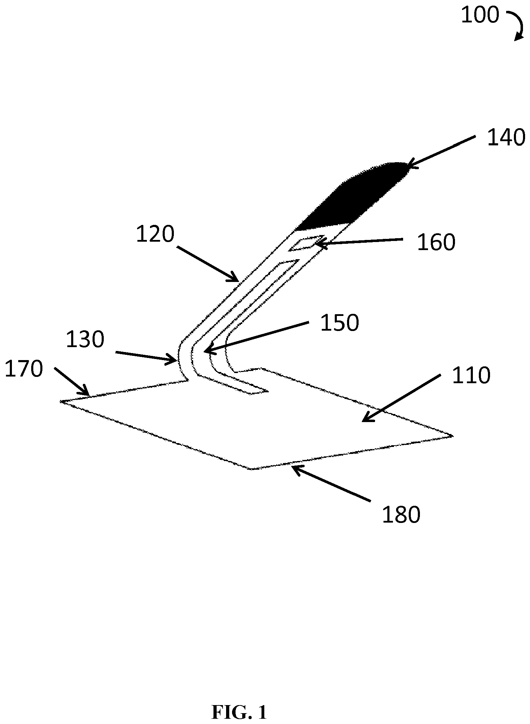

An implementation of a principal unit of the wound closure device of the present teachings is illustrated in **FIG. 1****.** Principal unit **100** of the wound closure device comprises base **110,** which has a top surface (shown in **FIG. 1**), a bottom surface opposite the top surface, inner edge **170,** outer edge **180,** and bridging link **120,** which is connected to base **110** at inner edge **170** by way of hinge region **130.** Bridging link **120** comprises tab **140** of appropriate size and shape as to allow for holding onto with at least two fingers. Hinge region **130** allows bridging link **120** to assume an extended (open) position, whereby bridging link **120** is substantially fully extended outwards and transversely with respect to inner edge **170;** and a closed position, whereby bridging link **120** is folded backwards over the top surface of base **110.** It should be clearly understood that bridging link **120** may be integrated with base **110,** either by way of fusion with base **110,** or by way of any other connection to base **110,** i.e. chemical, thermal or mechanical bonding or incorporation. It should also be understood that the sizes and shapes of various elements shown in **FIG. 1****,** as well as in other figures, are only illustrative and may vary, depending on specific implementations without departing from the general nature of the teachings.

The top surfaces of base **110** and bridging link **120** (all seen in **FIG. 1**), including hinge region **130** and tab **140,** are substantially free from adhesive. Whereas the bottom surface of base **110** (not shown) as well as the bottom surface of bridging link **120** (not shown), except for tab **140,** are substantially covered with adhesive which is strong enough as to ensure secure attachment of base **110** to human or animal skin while also allowing for removal of base **110** from skin if and when needed. In specific implementations it may also be desirable to leave hinge region **130** partially or completely free from adhesive, however not in the most general case contemplated herein.

It should be emphasized that one of the purposes of tab **140** is to allow for confident holding by a user of the device as to permit for easy manipulation of bridging link **120,** as well as other device manipulations. Therefore, any size and shape of tab **140,** while having it adhesive free as to ensure attaining holding and unhindered release thereof, is compatible with contemplated purposes of tab **140.**

For storage in the closed position, adhesive may optionally cover locking area **160** on the top surface of bridging link **120,** area **160** size is sufficient for securing bridging link in the folded back orientation, but may be expanded if greater tac is required. The adhesive used on locking area **160** may be the same adhesive applied elsewhere, e.g. to the bottom surface of base **110,** or it may be a different, for example a lighter tac adhesive. For storage, bridging link **120** is generally folded back onto the top surface of base **110,** and in case of utilizing locking area **160** the adhesive is allowed to attach to the top surface, thus securing bridging link **120** in the closed position.

Base **110** of unit **100** can be manufactured utilizing a variety of flexible or partially flexible, or unidirectionally flexible, or drapable materials. Bridging link **120,** including hinge region **130** and tabs **140,** may be made of the same material as base **110,** in which case a single piece, including **110, 120, 130** and **140,** is cut to size from a single sheet of the material selected. Alternatively, each element of the latter four elements, or any combination thereof, can be made of an individual material, or have individual additional elements. For example, in certain implementations hinge region **130** may comprise partially flexible component **150** which could promote stabilizing bridging link **120** in an intermediate angular position (illustrated in **FIG. 1**) between the extended (open) and the closed positions. However, the same stabilizing in the intermediate position effect can be achieved by choosing a partially flexible material for implementing any combination of the four elements that include hinge region **130.** Thus, in certain implementations, base **110** may be made of a flexible material, while bridging link **120,** including hinge region **130,** may be made of a partially flexible material, and bridging link **120** can be attached to base **110,** for example, by adhering it to the top surface of base **110,** or by other equivalent methods of attachment.

The flexible material of choice for manufacturing principal unit **100** may be a non-woven, spunbond nylon material, such as ORION® or PBN-11 fabric available from Cerex Advanced Fabrics, Inc. of Cantonment, Florida. In such case utilizing partially flexible component **150** is preferred, which may comprise various partially flexible polymers, e.g. polyamides, polyesters, perforated ethylene vinyl acetate material, thin metal or nylon wires, etc.

Principal unit **100** may also be manufactured utilizing various polyurethanes, or a breathable unidirectionally elastic substrate, as for base **110,** and bridging links **120,** including hinge region **130** and tab **140.** Utilizing a breathable unidirectionally elastic substrate allows for additional flexibility of unit **100,** which may be beneficial for certain applications. This flexibility, for example, can accommodate skin expansion or shrinkage in the process of wound healing. In such partially flexible component **150** may also be optionally implemented.

For some applications it may be desirable to manufacture principal unit **100** utilizing a material having hydrophobic properties as to be partially or completely fluid repellant. Such material choice would minimize absorption of bodily and other fluids into the principal unit **100.**

For some applications principal unit **100** may be manufactured utilizing a hook-and-loop fastener substrate, such as a low-profile VELCRO® fabric available from Velcro USA, Inc. of Manchester, New Hampshire. In this case, base **110** is manufactured of a loop tape material, having loops on the top surface of base **110** and adhesive of the loop-free bottom surface. Entire bridging link **120,** including hinge region **130** and tab **140,** is made of a hook tape material, having hooks on the bottom surface of bridging link **120** and thus no adhesive applied to the bridging link bottom surface. In this case utilizing partially flexible component **150** may not be necessary as hook tape itself is usually made of a partially flexible material, thus providing desirable properties in hinge region **130** of bridging link **120** as to stabilize bridging link **120** as an intermediate position. Bridging link **120** is attached to the top surface of base **110** by pressing its hooks against base **110** loops. For extra strength the attachment may be fortified by chemical bonding or thermal fusion. If necessary, smooth tab **140** on bridging link **120** can be implemented by melting down polymer hook tape hooks with local heating.

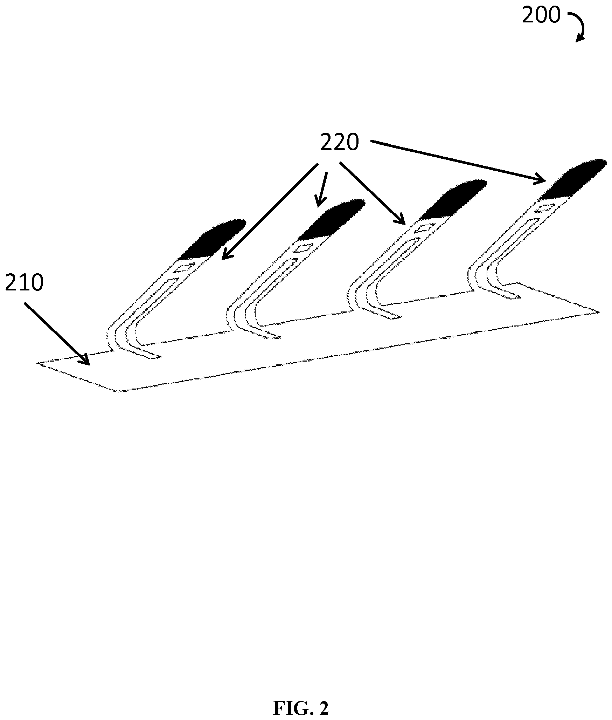

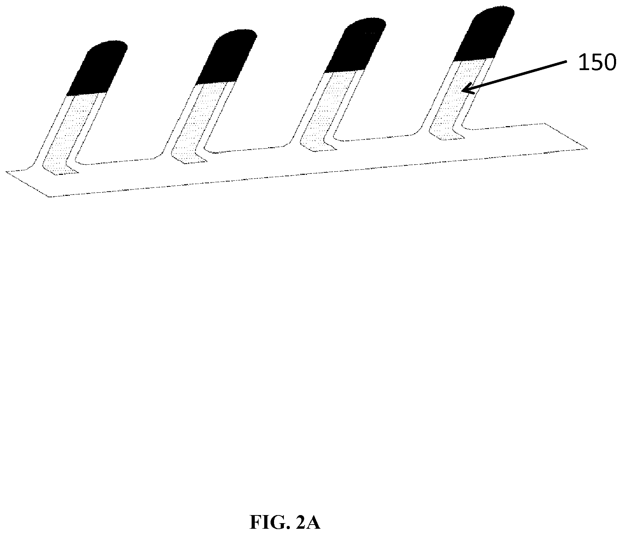

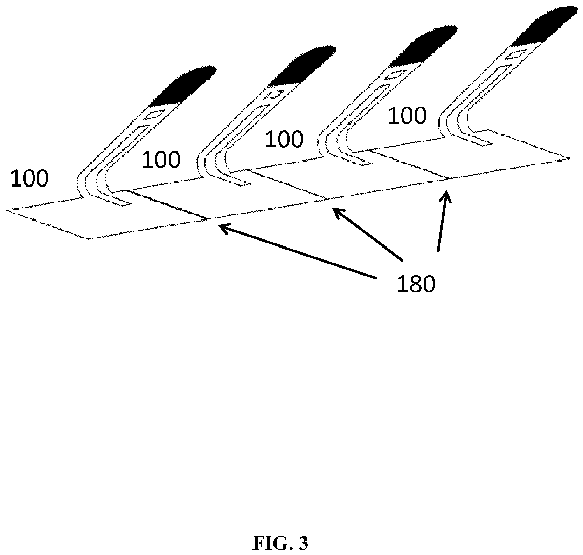

Consistent with the foregoing teachings, principal unit **100** can be realized in extended wound closure strip **200,** examples of which are shown in **FIG. 2****,** **FIG. 2A** and **FIG. 3****.** Wound closure strip **200** may be comprised of a continuous plurality of units **100** extending longitudinally, substantially along inner edge **170,** thus forming extended base **210** and a plurality of bridging links **220,** as shown in **FIG. 2****.** **FIG. 2A** illustrates an example of a wound closure strip comprised of principal units without a locking area and having extended partially flexible components **150.** As shown in **FIG. 3****,** in certain implementations it may be advantages to have perforation, colored, or shaded lines **180** on extended base **210,** for easy adjustments of base **210** length for particular application, or for enabling separation of wound closure strip **200** into individual units **100.**

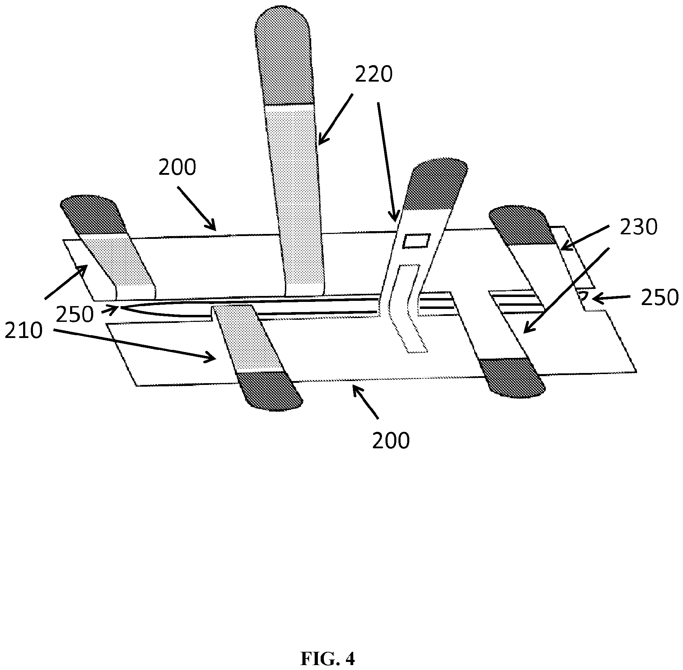

**FIG. 4** shows an example of two wound closure strips **200** applied to wound **250.** In the figure, one wound closure strip **200** is applied to one side of wound **250,** such that the inner edge of wound closure strip **200** base is aligned with one edge of the wound substantially along that edge of the wound and at a predetermined distance from the edge of the wound. Another wound closure strip **200** is applied to the opposite side of wound **250,** such that the inner edge of another wound closure strip **200** base is aligned with the opposite edge of the wound substantially along that edge of the wound and at another predetermined distance from the opposite edge of the wound. Application of the two wound closure strips **200** is done in such a manner as to have bridging links of the one wound closure strip **200** offset or facing the spaces between bridging links of the other wound closure strip **200,** as illustrated in **FIG. 4****.** Initially, the offset bridging links, facing each other, of wound closure strips **200** are in closed positions **210.** For closing wound **250** the bridging links are brought into extended positions **230,** when the bridging links of one wound closure strip **200** are attached to the top surface of the opposite wound closure strip **200** via the adhesive on the bottom surface of the bridging links. A complete closure of wound **250** may be achieved by bringing all bridging links into extended positions **230.**

Wound closure can be readjusted, and if necessary improved, by detaching opposing bridging links from the opposite base and reattaching the bridging links while pulling the opposing bridging links into opposite direction, essentially in a "shoe string" manner. Tabs **140** on bridging links **120** (see **FIG. 1**) are especially useful for such detaching and reattaching of the bridging links. Before attaching bridging links for closing the wound, the bridging links can be stabilized in the intermediate position **220** (as shown in **FIG. 4**). This intermediate position is accomplished due to the presence of partially flexible materials in their hinge regions. Having the bridging links stabilized in intermediate positions **220** is beneficial because this allows for unhindered wound manipulation when the wound is completely or partially open for readjusting the wound closure, or for intermediate cleaning of the wound from exudates, applying medications to the wound, and/or other wound manipulations. Further, while in intermediate positions **220,** the bridging links are better presented for handling by the user, especially considering that practitioners, who are most likely to use the devices of the present teachings, are routinely wearing surgical gloves. Thus, enabling stable orientation of bridging links in intermediate positions **220** also enables easier wound manipulation by a practitioner.

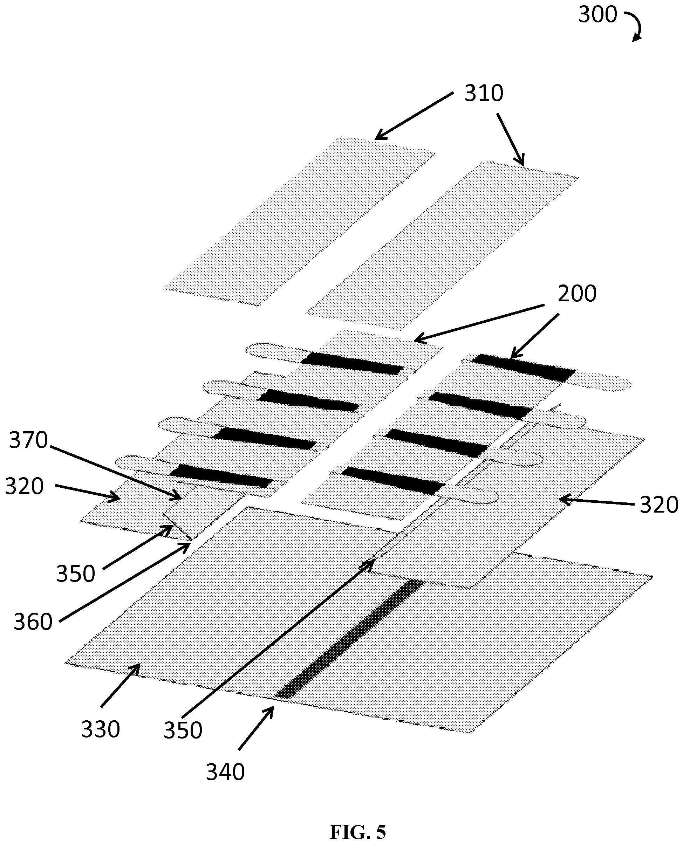

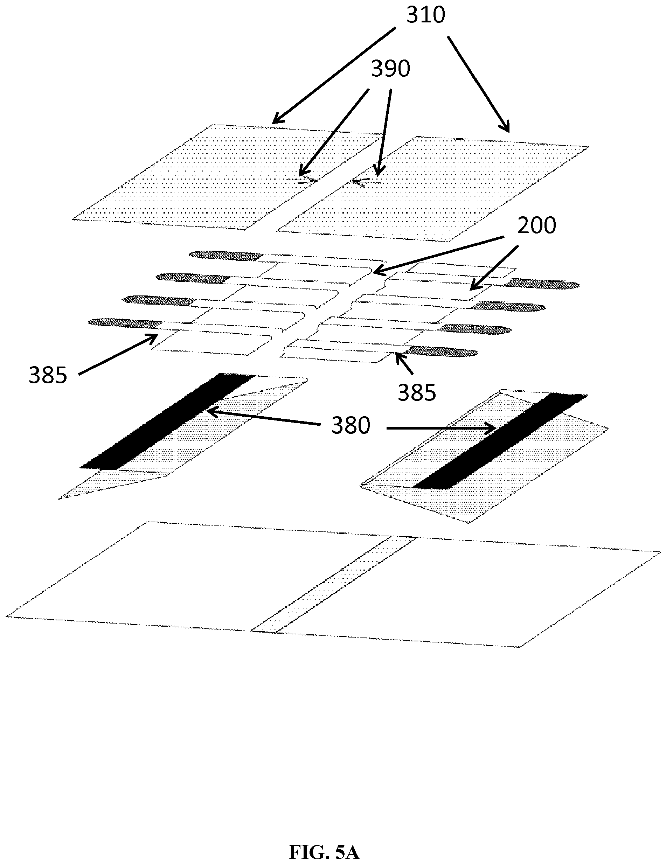

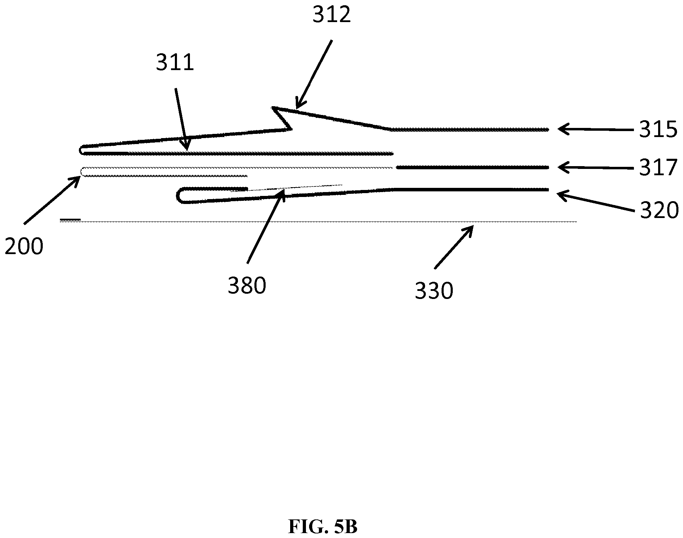

An exploded view of an example of an assembled ready-for-use wound closure device of the present teachings is illustrated in **FIG. 5****.** Wound closure device **300** is comprised of two wound closure strips **200** assembled facing each other on support film **330.** A colored or shaded line **340** on support film **330** may be used to indicate to user the wound direction. Portions of base bottom surfaces of two wound closure strips **200** are covered by holding films **320.** Each holding film **320** has a folded lip **350.** Each folded lip **350** is folded along lip folding line **360** and has lip outer edge **370.** Two wound closure strips **200** are attached with portions of their base bottom surfaces, not covered by holding films **320,** to support film **330** so as to have their inner edges facing each other and line **340** and at a predetermined distance from line **340,** as illustratively shown in **FIG. 5****.** In an assembled ready-for-use device, bridging links of each wound closure strip **200** are in closed positions, folded over backwards. Hinge regions of one wound closure strip **200** are facing into spaced between hinge regions of the opposite wound closure strip **200,** as they would be when the device is applied to a wound. A portion of each wound closure strip **200** base bottom surface is covered with folded lip **350** of holding film **320,** which may be clear or color coded, the remainder of holding film **320** extending beyond base outer edge as to cover at least a portion of bridging links' tabs extending beyond base outer edge. In some, but not all, implementations of the wound closure device the tabs begin at the base outer edge when bridging links are in the closed (folded backwards) position. Holding films **320** are applied to the base bottom surface of wound closure strips **200** such as to leave uncovered a portion of the base bottom surface adjacent to each base inner edge. This is achieved by placing lip outer edges **370** at a predetermined distance from base inner edges, thus leaving portions of base bottom surfaces uncovered with lips **350.** The adhesive on these uncovered surfaces are used to attach wound closure strips **200** to support film **330.** The top of the assembled ready-for-use wound closure device **300** is protected with clear or color coded top films **310** covering each individual wound closure strip **200** of the device by attaching to the adhesive on the bottom surfaces of the folded backwards bridging links.

Folded lips **350** may be made wide enough as to allow lip outer edges **370** to protrude a predetermined distance beyond base outer edges when holding strips **320** are attached to wound closure strips **200.** As shown in **Fig. 5A****,** the surfaces (facing bases of wound closure strips **200**) of the protruding portions **380** of folded lips **320** may be covered with adhesive for adhering to portions of bridging links **385** protruding beyond base outer edges of wound closure strips **200** when the bridging links are in closed positions,. In this case having adhesive in locking area **160** of the bridging links (see **Fig. 1**) may not be necessary as the bridging links will be held in closed positions by adhering to the protruding portions **380** adjacent to outer edges **370** of folded lips **350** when holding strips **320** are attached to wound closure strips **200.** In the example illustrated in **FIG. 5A****,** color coded top films **310** covering each individual wound closure strip **200** have arrow markings **390,** which may be provided for helping in aligning opposing wound closure strips while placing the on opposing sides of the wound prior to removing films **310.**

In certain applications it is desirable to have a wound closure device having top film **310** unified with holding film **320** such that both can be removed together. **FIG. 5B** illustrates a cross section of an example of an assembled ready-for-use wound closure device (only a half id shown) in which a top film and a holding film are interconnected. In the example shown, as in the previous example illustrated in **FIG. 5A****,** wound closure strip **200,** having no adhesive in locking areas is assembled on support film **330,** sandwiched between holding film **320,** having adhesive extended area **380** for holding bridging links in closed position, and extended top film **315.** Top film **315** comprises a closure strip protective portion **311,** which is functionally equivalent to top film **310** in the previous example. Extended top film **315** folds over as to provide sufficient surface are to attach to an adhesive covered top portion of connecting film **317,** as illustrated in **FIG. 5B****.** Opposite adhesive covered bottom portion of connecting film **317** is attached to a protruding portion of holding film **320,** thus unifying films **315** and **320.** Extended top film **315** may optionally comprise delay fold **312.**

Wound closure device **300** can be used as follows. Initially, one wound closure strip **200** covered with holding film **320** and top film **310** is removed from support film **330.** This can be accomplished while holding onto the surfaces of films **320** and **310** to avoid adhesive covered areas of wound closure strip **200.** Continuing to hold the assembly of wound closure strip **200** with films **320** and **310** after removing wound closure strip **200** from support film **330,** wound closure strip **200** is applied to one side of the wound being closed with the exposed adhesive of the unprotected portion of the base adjacent to the base inner edge, leaving a predetermined distance between the edge of the wound and the base inner edge of wound closure strip **200** (as illustratively shown in **FIG. 4**). Because only a limited adhesive covered portion of the base of wound closure strip **200** is exposed, the rest being covered by holding film **320,** it is easy to follow the edge of the wound with the base outer edge of wound closure strip **200** in case of a complex nature of the wound. If necessary, such as with an irregularly curved wound, the exposed base inner edge can be intermittently detached from skin and reattached back after a position adjustment. In certain applications, it may be desirable to remove top film **310** prior to applying the base inner edge of wound closure strip **200** to skin. In this case, only holding film **320,** with protruding onto it portions of bridging links adhesive free tabs, is used for holding wound closure strip **200** while applying it to skin. After its base outer edge has been completely applied, holding film **320** is removed from under wound closure strip **200** and the remaining exposed adhesive on its base is allowed to attach to skin. After one wound closure strip **200** has been applied to one side of the wound, the other wound closure strip **200** is removed from support film **330** and applied to the opposite side of the wound in a similar fashion. During application, the two opposing wound closure strips **200** are aligned essentially as shown in **FIG. 4****.** After both wound closure strips **200** have been securely attached to skin on the opposite sides of the wound, their bridging linked are detached from their closed positions, while holding and pulling them by their tabs (in case of adhesive covered protruding areas of folded lips **350** bridging links are released when holding film **320** is removed). The wound is closed by attaching the bridging links to the base top surfaces of the opposing wound closure strips **200,** essentially in a "shoe string" manner. If it is necessary to adjust wound closure, any number of bridging links can be intermittently detached and held stably in intermediate position **220** without hindering access to the wound or neighboring bridging links, as illustrated in **FIG. 4****,** until reattached later.

The implementation of the device illustrated in **FIG. 5B** can be applied as follows. Initially, one wound closure strip **200** covered with unified holding film **320** and top film **315** is removed from support film **330.** This can be accomplished while holding onto the surfaces of films **320** and **315** to avoid adhesive covered areas of wound closure strip **200.** Continuing to hold the assembly of wound closure strip **200** with films **320** and **315** after removing wound closure strip **200** from support film **330,** wound closure strip **200** is applied to one side of the wound as explained previously. After one wound closure strip **200** has been applied to one side of the wound, the other wound closure strip **200** is removed from support film **330** and applied to the opposite side of the wound in a similar fashion as explained previously. Subsequently, unified films **320** and **315** are removed by pulling in a direction away from the wound in s single smooth motion. Optional delay fold **312** assists in providing for detaching film **320** from the bottom of strip **200** prior to detaching portion **311** from the top of strip **200.** After removing films **320** and **315** the bridging links of the device can be utilized to close the wound as previously described.

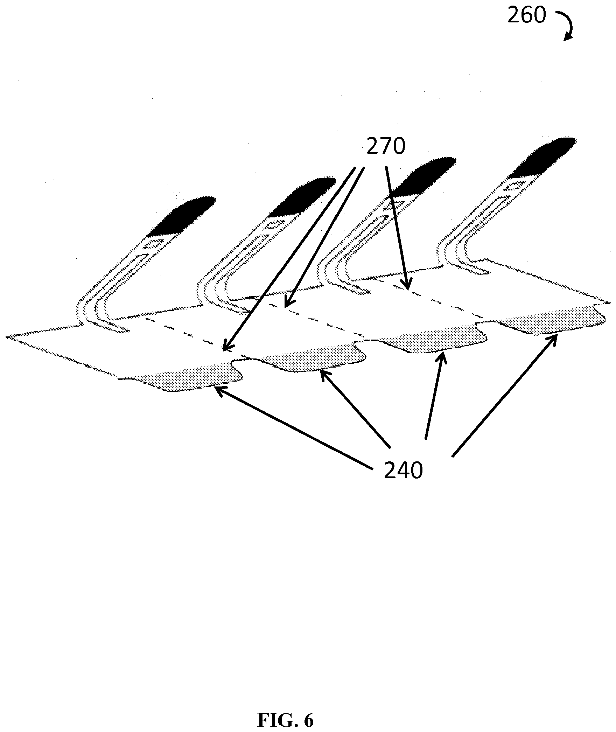

In certain implementations it may be desirable to have adhesive free holding tabs extensions on the base outer side of wound closure strips, as illustrated with color code or noncoded holding tabs **240** in **FIG. 6****.** Holding tabs **240** may be used without holding film **320,** in which case holding film **320** is not included into assembled ready-for-use wound closure device, or in combination with holding film **320.** For example, holding tabs **240** may be useful for holding while separating wound closure strip **260** into shorter units along perforation lines **270,** as illustrated in **FIG. 6****.**

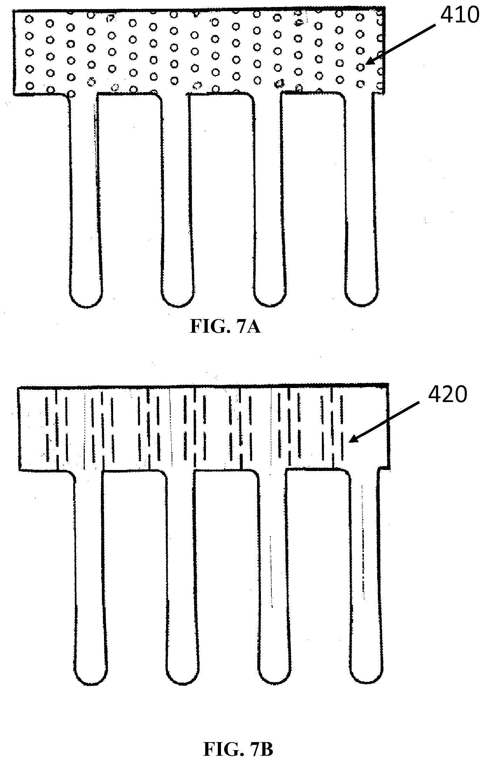

Referring to **FIG. 7****,** in certain applications it may be advantageous to implement openings of various shapes on wound closure strip base as to allow the base to stretch or shrink in the course of wound healing, thereby protecting sensitive/vulnerable skin from irritation, blistering or sheer. For example, such openings may be implemented as holes **410,** as illustrated in **FIG. 7A****,** or slits **420,** as illustrated in **FIG. 7B****.** Other opening shapes may be used as appropriate.

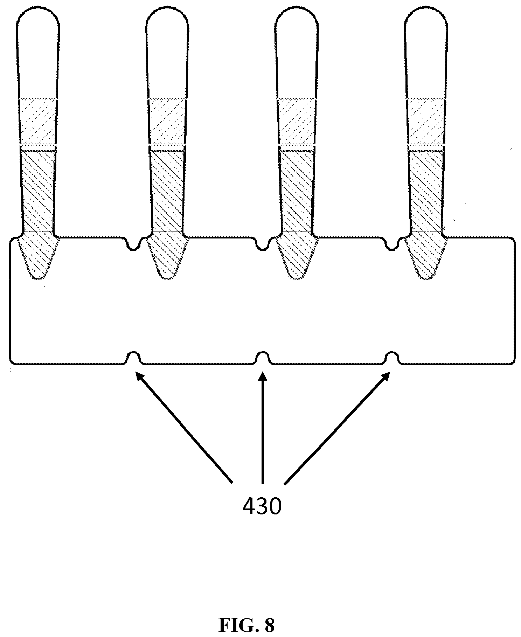

Referring to **FIG. 8****,** in certain applications it may be advantageous to implement notches **430** on wound closure strip base as to augment, for example, separating the strip into individual units or adjusting the length of the base. Notches may also be useful in aligning opposing strips with respect to each other when applying the strips to a wound. FIG. 8 shows a U-shaped implementation of the notches, which among other advantages may be advantageous to simplify strip manufacturing. However, other shapes of the notches may also be desirable for difference applications.

In certain embodiments, the closure device **100** may be formed of a conformable non-woven textile-like fabric, e.g., formed of nylon or other synthetic material. The closure device **100** is preferably formed of ORION® spun bond nylon fabric. In particularly preferred embodiments, the closure device **100** is formed of having a weight in the range of from about 1 to about 2 ounces per square yard and is most preferably in the range of from about 1.8 to about 2.0 ounces per square yard.

The spunbond fabric is breathable, translucent, and high-strength with longitudinal flexibility. The material is formed of nylon and is adaptable to a variety of environments. The flexibility and weight of the material allows it to curve around wounds in a one piece construction. Also, other fabrics that have longitudal, length flex and less width flex or cross directional flex provide a useful fabric for this invention. Other devices are inflexible and need to be cut into separately applied segments in order to contour to a curved or other nonlinear or irregular wound.

The invention has been described with reference to the preferred embodiments. Modifications and alterations will occur to others upon reading and understanding the preceding detailed description. It is intended that the invention be construed as encompassing all such modifications and alterations insofar as they come within the scope of the appended claims or the equivalents thereof.

## Claims

1. A device (100,200) for closing a wound, comprising:
a flexible base strip (110,210) having a bottom surface coated with an adhesive to adhere to one side of a wound, a top surface opposite the bottom surface, an inner edge and an outer edge;
a plurality of bridging links (120,220) integrated with the base strip in axially-spaced relation, said bridging links extending transversely from said inner edge;
each of said bridging links movable about a hinge region (130) between a first, storage position wherein said bridging links are folded over the top surface of the base strip and a second, extended position wherein said bridging links extend outwardly from said inner edge;
said bridging links each having a first, engaging surface and a second surface opposite the engaging surface, said first engaging surface having an attachment region for attaching to another top surface of another base strip when said another base strip is placed on an opposite side of the wound and the bridging links are in said extended position; **characterised in that**
said hinge region comprises a partially flexible material (150) capable of stabilising the orientation of said bridging links in an intermediate angular position between said first and said second positions.

2. The device of claim 1, further comprising a holding tab (240) integrated with said outer edge of the base strip and extending outwardly a predetermined distance from said outer edge;
wherein said holding tab is substantially free from adhesive; and
wherein said holding tab may be used for holding.

3. The device of claim 1, wherein:
said flexible base strip comprises a loop tape of a hook-and-loop fastener substrate, said loop tape presenting loops at least on a portion of said top surface; and
said bridging links comprise a hook tape of said hook-and-loop fastener substrate, said hook tape presenting hooks in said attachment region.

4. The device for closing a wound of claim 1, wherein each of said bridging links further comprises
(a) a pulling tab (140) at its outer end; wherein said pulling tab is substantially free from adhesive; and
wherein said pulling tab may be used for holding; or
(b) adhesive in a locking area (160) on said second surface to releasably secure said second surface to the top surface of the base strip when the bridging links are in the storage position.

5. The device of claim 1, wherein said base strip and said bridging links comprise polyurethane or a breathable unidirectionally elastic substrate.

6. The device of claim 1, further comprising
(a) transverse indicia on said top surface or said bottom surface of said base strip, wherein said transverse indicia may be used for guiding separating said base strip into shorter fragments; or
(b) a holding film (320) attached to said bottom surface of said base strip at a predetermined distance from said inner edge of said base strip and extending beyond said outer edge of said base strip.

7. The device of claim, 1 further comprising:
a first closure strip removably attached to a lower support sheet (330);
a second closure strip removably attached to the lower support sheet, the first and second closure strips in aligned, facing relation;
each of said first and second closure strips including one of the flexible base strip.

8. The device for closing a wound of claim 7, wherein each of said bridging links further comprises a pulling tab (140) at its outer end; wherein said pulling tab is substantially free from adhesive; and
wherein said pulling tab may be used for holding.

9. The device of claim 7, wherein said base strip and said bridging links comprise polyurethane or a breathable unidirectionally elastic substrate.

10. The device of claim 7, wherein each of said bridging links further comprises adhesive in a locking area (160) on said second surface to releasably secure said second surface to the top surface of the base strip when the bridging links are in the storage position.

11. The device of claim 7, further comprising transverse indicia on said top surface or said bottom surface of said first and second closure strips, wherein said transverse indicia may be used for guiding separating said first and second closure strips into shorter fragments.

12. The device of claim 7, further comprising:
a first holding film (320) removably attached to said bottom surface of said first closure strip, between said first closure strip and said support sheet, at a predetermined distance from said inner edge of said first closure strip and extending beyond said outer edge of said first closure strip; and
a second holding film (320) removably attached to said bottom surface of said second closure strip, between said second closure strip and said support sheet, at a predetermined distance from said inner edge of said second closure strip and extending beyond said outer edge of said second closure strip; and, optionally,
wherein portions of the first holding film and said second holding film extending beyond said outer edge of said first and second closure strips are covered with adhesive configured to removably adhere to said bridging links when said bridging links are in said first storage position.

13. The device of claim 7, further comprising a top film (310) removably attached to said attachment region of at least one of said bridging links.

14. The device of claim 7, wherein said support sheet further comprises an indicia line between said first and second closure strips.

## Patentansprüche

1. Vorrichtung (100, 200) zum Schließen einer Wunde, die Folgendes umfasst:
einen biegsamen Grundstreifen (110, 210), mit einer unteren Oberfläche, die mit einem Klebstoff beschichtet ist, um an einer Seite einer Wunde zu kleben, einer der unteren Oberfläche gegenüberliegenden oberen Oberfläche, einem inneren Rand und einem äußeren Rand;
eine Vielzahl von Brückengliedern (120, 220), die in axial beabstandeter Beziehung in dem Grundstreifen integriert sind, wobei sich die Brückenglieder in Querrichtung von dem inneren Rand erstrecken;
wobei die Brückenglieder jeweils um eine Gelenkregion (130) zwischen einer ersten, einer Aufbewahrungsstellung, in der die Brückenglieder auf die obere Oberfläche des Grundstreifens geklappt sind, und einer zweiten, ausgeklappten Stellung, in der sich die Brückenglieder von dem inneren Rand nach außen erstrecken, beweglich sind;
wobei die Brückenglieder jeweils eine erste, eine Eingriffsoberfläche, und eine der Eingriffsoberfläche gegenüberliegende zweite Oberfläche aufweisen, wobei die erste, die Eingriffsoberfläche eine Befestigungsregion zum Befestigen an einer anderen oberen Oberfläche eines anderen Grundstreifens, wenn der andere Grundstreifen auf einer gegenüberliegende Seite der Wunde platziert ist und sich die Brückenglieder in der ausgeklappten Stellung befinden, aufweist; **dadurch gekennzeichnet, dass**
die Gelenkregion ein teilweise biegsames Material (150) umfasst, das in der Lage ist, die Orientierung der Brückenglieder in einer mittleren Winkelstellung zwischen der ersten und der zweiten Stellung zu stabilisieren.

2. Vorrichtung nach Anspruch 1, weiter umfassend eine Haltelasche (240), die in den äußeren Rand des Grundstreifens integriert ist und sich um eine vorbestimmte Entfernung von dem äußeren Rand nach außen erstreckt;
wobei die Haltelasche im Wesentlichen frei von Klebstoff ist; und
wobei die Haltelasche zum Halten verwendet werden kann.

3. Vorrichtung nach Anspruch 1, wobei:
der biegsame Grundstreifen ein Schlingenband eines Klettverschlusssubstrats umfasst, wobei das Schlingenband Schlingen an mindestens einem Abschnitt der oberen Oberfläche darbietet; und
die Brückenglieder ein Hakenband des Klettverschlusssubstrats umfassen, wobei das Hakenband Haken in der Befestigungsregion darbietet.

4. Vorrichtung zum Schließen einer Wunde nach Anspruch 1, wobei die Brückenglieder jeweils weiter Folgendes umfassen:
(a) eine Zuglasche (140) an ihrem äußeren Ende;
wobei die Zuglasche im Wesentlichen frei von Klebstoff ist; und
wobei die Zuglasche zum Halten verwendet werden kann; oder
(b) Klebstoff in einem Arretierbereich (160) auf der zweiten Oberfläche, um die zweite Oberfläche lösbar an der oberen Oberfläche des Grundstreifens zu fixieren, wenn sich die Brückenglieder in der Aufbewahrungsstellung befinden.

5. Vorrichtung nach Anspruch 1, wobei der Grundstreifen und die Brückenglieder Polyurethan oder ein atmungsfähiges unidirektional elastisches Substrat umfassen.

6. Vorrichtung nach Anspruch 1, die weiter Folgendes umfasst:
(a) querlaufendende Markierungen auf der oberen Oberfläche oder der unteren Oberfläche des Grundstreifens, wobei die querlaufenden Markierungen zum Führen der Trennung des Grundstreifens in kürzere Fragmente verwendet werden können, oder
(b) einen Haltefilm (320), der an der unteren Oberfläche des Grundstreifens in einer vorbestimmten Entfernung von dem inneren Rand des Grundstreifens angebracht ist und sich über den äußeren Rand des Grundstreifens hinaus erstreckt.

7. Vorrichtung nach Anspruch 1, die weiter Folgendes umfasst:
einen ersten Verschlussstreifen, der abnehmbar an einer unteren Trägerfolie (330) angebracht ist;
einen zweiten Verschlussstreifen, der abnehmbar an der unteren Trägerfolie angebracht ist, wobei sich der erste und der zweite Verschlussstreifen in einer aufeinander ausgerichteten, einander zugewandten Beziehung befinden;
wobei der erste und der zweite Verschlussstreifen jeweils einen des biegsamen Grundstreifens umfassen.

8. Vorrichtung zum Schließen einer Wunde nach Anspruch 7, wobei die Brückenglieder weiter jeweils eine Zuglasche (140) an ihrem äußeren Ende umfassen;
wobei die Zuglasche im Wesentlichen frei von Klebstoff ist; und
wobei die Zuglasche zum Halten verwendet werden kann.

9. Vorrichtung nach Anspruch 7, wobei der Grundstreifen und die Brückenglieder Polyurethan oder ein atmungsfähiges unidirektional elastisches Substrat umfassen.

10. Vorrichtung nach Anspruch 7, wobei die Brückenglieder weiter jeweils Klebstoff in einem Arretierbereich (160) auf der zweiten Oberfläche umfassen, um die zweite Oberfläche lösbar an der oberen Oberfläche des Grundstreifens zu fixieren, wenn sich die Brückenglieder in der Aufbewahrungsstellung befinden.

11. Vorrichtung nach Anspruch 7, weiter umfassend querlaufende Markierungen auf der oberen Oberfläche oder der unteren Oberfläche des ersten und des zweiten Verschlussstreifens, wobei die querlaufenden Markierungen zum Führen der Trennung des ersten und des zweiten Verschlussstreifens in kürzere Fragmente verwendet werden können.

12. Vorrichtung nach Anspruch 7, die weiter Folgendes umfasst:
einen ersten Haltefilm (320), der zwischen dem ersten Verschlussstreifen und der Trägerfolie in einer vorbestimmten Entfernung von dem inneren Rand des ersten Verschlussstreifens abnehmbar an der unteren Oberfläche des ersten Verschlussstreifens angebracht ist und sich über den äußeren Rand des ersten Verschlussstreifens hinaus erstreckt; und
einen zweiten Haltefilm (320), der zwischen dem zweiten Verschlussstreifen und der Trägerfolie in einer vorbestimmten Entfernung von dem inneren Rand des zweiten Verschlussstreifens abnehmbar an der unteren Oberfläche des zweiten Verschlussstreifens angebracht ist und sich über den äußeren Rand des zweiten Verschlussstreifens hinaus erstreckt; und optional
wobei sich über den äußeren Rand des ersten und des zweiten Verschlussstreifens hinaus erstreckende Abschnitte des ersten Haltefilms und des zweiten Haltefilms mit Klebstoff bedeckt sind, der dazu konfiguriert ist, abnehmbar an den Brückengliedern zu kleben, wenn sich die Brückenglieder in der ersten, der Aufbewahrungsstellung befinden.

13. Vorrichtung nach Anspruch 7, weiter umfassend einen Deckfilm (310), der abnehmbar an der Befestigungsregion mindestens eines der Brückenglieder angebracht ist.

14. Vorrichtung nach Anspruch 7, wobei die Trägerfolie weiter eine Markierungslinie zwischen dem ersten und dem zweiten Verschlussstreifen umfasst.

## Revendications

1. Dispositif (100, 200) servant à fermer une plaie, comportant :
une bande de base flexible (110, 210) ayant une surface inférieure enduite d'un adhésif pour adhérer sur un côté d'une plaie, une surface supérieure à l'opposé de la surface inférieure, un bord intérieur et un bord extérieur ;
une pluralité de liaisons de rapprochement (120, 220) intégrées à la bande de base selon une relation espacée dans le sens axial, lesdites liaisons de rapprochement s'étendant dans le sens transversal depuis ledit bord intérieur ;
chacune desdites liaisons de rapprochement étant mobile au niveau d'une région formant charnière (130) entre une première position de conditionnement dans laquelle lesdites liaisons de rapprochement sont pliées sur la surface supérieure de la bande de base et une deuxième position déployée dans laquelle lesdites liaisons de rapprochement s'étendent vers l'extérieur depuis ledit bord intérieur ;
lesdites liaisons de rapprochement ayant chacune une première surface de mise en prise et une deuxième surface à l'opposé de la surface de mise en prise, ladite première surface de mise en prise ayant une région d'attache servant à des fins d'attache sur une autre surface supérieure d'une autre bande de base quand ladite une autre bande de base est placée sur un côté opposé de la plaie et les liaisons de rapprochement sont dans ladite position déployée ; **caractérisé en ce que**
ladite région formant charnière comporte un matériau partiellement flexible (150) en mesure de stabiliser l'orientation desdites liaisons de rapprochement dans une position angulaire intermédiaire entre lesdites première et deuxième positions.

2. Dispositif selon la revendication 1, comportant par ailleurs une languette de retenue (240) intégrée audit bord extérieur de la bande de base et s'étendant vers l'extérieur sur une distance prédéterminée depuis ledit bord extérieur ;
dans lequel ladite languette de retenue est sensiblement exempte de tout adhésif ; et
dans lequel ladite languette de retenue peut être utilisée à des fins de retenue.

3. Dispositif selon la revendication 1, dans lequel :
ladite bande de base flexible comporte un ruban à boucles d'un substrat de fermeture à boucles et à crochets, ledit ruban à boucles présentant des boucles au moins sur une partie de ladite surface supérieure ; et
lesdites liaisons de rapprochement comportent un ruban à crochets dudit substrat de fermeture à boucles et à crochets, ledit ruban à crochets présentant des crochets dans ladite région d'attache.

4. Dispositif servant à fermer une plaie selon la revendication 1, dans lequel chacune desdites liaisons de rapprochement comporte par ailleurs
(a) une languette à tirer (140) au niveau de son extrémité extérieure ;
dans lequel ladite languette à tirer est sensiblement exempte de tout adhésif ; et
dans lequel ladite languette à tirer peut être utilisée à des fins de retenue ; ou
(b) de l'adhésif dans une zone de verrouillage (160) sur ladite deuxième surface à des fins d'assujettissement libérable de ladite deuxième surface sur la surface supérieure de la bande de base quand les liaisons de rapprochement sont dans la position de conditionnement.

5. Dispositif selon la revendication 1, dans lequel ladite bande de base et lesdites liaisons de rapprochement comportent du polyuréthanne ou un substrat à élasticité unidirectionnelle et perméable à l'air.

6. Dispositif selon la revendication 1, comportant par ailleurs
(a) des repères transversaux sur ladite surface supérieure ou sur ladite surface inférieure de ladite bande de base, dans lequel lesdits repères transversaux peuvent être utilisés pour guider la séparation de ladite bande de base en des fragments plus courts ; ou
(b) un film de retenue (320) attaché sur ladite surface inférieure de ladite bande de base à une distance prédéterminée depuis ledit bord intérieur de ladite bande de base et s'étendant au-delà dudit bord extérieur de ladite bande de base.

7. Dispositif selon la revendication 1, comportant par ailleurs :
une première bande de fermeture attachée de manière amovible sur une feuille de support inférieure (330) ;
une deuxième bande de fermeture attachée de manière amovible sur la feuille de support inférieure, les première et deuxième bandes de fermeture étant en une relation se faisant face l'une vers l'autre de manière alignée ;
chacune desdites première et deuxième bandes de fermeture comprenant l'une de la bande de base flexible.

8. Dispositif servant à fermer une plaie selon la revendication 7, dans lequel chacune desdites liaisons de rapprochement comporte par ailleurs une languette à tirer (140) au niveau de son extrémité extérieure ;
dans lequel ladite languette à tirer est sensiblement exempte de tout adhésif ; et
dans lequel ladite languette à tirer peut être utilisée à des fins de retenue.

9. Dispositif selon la revendication 7, dans lequel ladite bande de base et lesdites liaisons de rapprochement comportent du polyuréthanne ou un substrat à élasticité unidirectionnelle et perméable à l'air.

10. Dispositif selon la revendication 7, dans lequel chacune desdites liaisons de rapprochement comporte par ailleurs de l'adhésif dans une zone de verrouillage (160) sur ladite deuxième surface à des fins d'assujettissement libérable de ladite deuxième surface sur la surface supérieure de la bande de base quand les liaisons de rapprochement sont dans la position de conditionnement.

11. Dispositif selon la revendication 7, comportant par ailleurs des repères transversaux sur ladite surface supérieure ou sur ladite surface inférieure desdites première et deuxième bandes de fermeture, dans lequel lesdits repères transversaux peuvent être utilisés pour guider la séparation desdites première et deuxième bandes de fermeture en des fragments plus courts.

12. Dispositif selon la revendication 7, comportant par ailleurs :
un premier film de retenue (320) attaché de manière amovible sur ladite surface inférieure de ladite première bande de fermeture, entre ladite première bande de fermeture et ladite feuille de support, à une distance prédéterminée depuis ledit bord intérieur de ladite première bande de fermeture et s'étendant au-delà dudit bord extérieur de ladite première bande de fermeture ; et
un deuxième film de retenue (320) attaché de manière amovible sur ladite surface inférieure de ladite deuxième bande de fermeture, entre ladite deuxième bande de fermeture et ladite feuille de support, à une distance prédéterminée depuis ledit bord intérieur de ladite deuxième bande de fermeture et s'étendant au-delà dudit bord extérieur de ladite deuxième bande de fermeture ; et, éventuellement,
dans lequel des parties du premier film de retenue et dudit deuxième film de retenue s'étendant au-delà dudit bord extérieur desdites première et deuxième bandes de fermeture sont recouvertes d'un adhésif configuré pour adhérer de manière amovible sur lesdites liaisons de rapprochement quand lesdites liaisons de rapprochement sont dans ladite première position de conditionnement.

13. Dispositif selon la revendication 7, comportant par ailleurs un film supérieur (310) attaché de manière amovible à ladite région d'attache d'au moins l'une desdites liaisons de rapprochement.

14. Dispositif selon la revendication 7, dans lequel ladite feuille de support comporte par ailleurs une ligne de repères entre lesdites première et deuxième bandes de fermeture.
